# EUROPEAN PATENT APPLICATION

(11) **EP 4 201 339 A1**
(43) Date of publication of application: **28.06.2023**
(21) Application number: 21857370.7
(22) Date of filing: 30.06.2021
(51) Int. Cl.: A61B 8/00

(54) **MUSCLE TRAINING METHOD AND SYSTEM FOR PROVIDING VISUAL FEEDBACK BY USING ULTRASONIC IMAGING**

(30) Priority: 21.08.2020 CN 202010854798
(71) Applicant: Eieling Technology Limited, Hong Kong (HK)
(72) Inventor: HUANG, Zihao, Hong Kong Science Park, NT Hong Kong (CN); WANG, Like, Hong Kong Science Park, NT Hong Kong (CN); ZHENG, Yongping, Hong Kong Science Park, NT Hong Kong (CN)
(74) Representative: Puschmann Borchert Kaiser Klettner Patentanwälte Partnerschaft mbB
(86) International application number: PCT/CN2021/103783
(87) International publication number: WO 2022/037274

(57) **Abstract**

A muscle training method and system for providing visual feedback by using ultrasonic imaging. The method comprises: on the basis of a preset ultrasonic device, determining a training object and target training data (S101); acquiring ultrasonic images of a target muscle of the training object during contraction process, and respectively selecting regions of interest from ultrasonic image frames (S 102); extracting muscle characteristic parameters of the target muscle from the selected regions of interest (S103a); according to the muscle characteristic parameters, obtaining a training index value, representing the degree of activation of the target muscle of the training object during the exercise (S103b); converting the training index value into visual feedback signals displayed in real time, and synchronously displaying the visual feedback signal and the ultrasonic images (S104a); comparing the training index value with the target training data, and according to an instantaneous comparison result between the training index value and the target training data, generating prompt information corresponding to the target muscle so as to guide the training object in real time to perform target muscle training (S104b). Thus, a user can be guided to complete a targeted muscle activation task.

## Description

### TECHNICALFIELD

The present application relates to the field of muscle training, and more specially relates to a muscle training method and system for providing visual feedback by using ultrasonic imaging.

### BACKGROUND

For the field of sports medicine, rehabilitation medicine and fitness science, how to effectively control the movement of target muscles to achieve specific training purposes has significance and value. For example, there is a close relationship between liver health and muscle training. Taking fatty liver as an example, studies have shown that the degree of fat accumulation in the liver is related to body weight. When the body fat of obese people is controlled, generally, the infiltration of fat in their liver is also reduced or disappeared. The management and rehabilitation of chronic liver diseases, such as liver fibrosis and fatty liver, usually includes muscle training.

Generally speaking, traditional muscle training is empirically oriented and has low reliability. Trainers can only rely on subjective judgment and oral instructions to guide trainees and affect the training quality. And the traditional muscle training can not directly feedback the training performance and training effect to users at the first time, which is not conducive to user experience.

The above content is only used to help understand the technical solution of the present application, and does not mean that the above content is recognized as the prior art.

### SUMMARY

The main purpose of the present application is to provide a muscle training method and system for providing visual feedback by using ultrasonic imaging, and to solve the problem of how to intuitively feed back the training performance and training effect to users at the first time in the muscle training process to improve the user experience.

The present application provides a muscle training method for providing visual feedback by using ultrasonic imaging, wherein, the method comprises:
on the basis of a preset ultrasonic device, determining a training object and target training data and associating the target training data with the training object;
acquiring ultrasonic images of a target muscle of the training object during contraction process, and respectively selecting regions of interest from ultrasonic image frames;
extracting muscle characteristic parameters of the target muscle from the selected regions of interest;
according to the muscle characteristic parameters, obtaining a training index value, representing a degree of activation of the target muscle of the training object during the exercise;
converting the training index value into visual feedback signals displayed in real time, and synchronously displaying the visual feedback signal and the ultrasonic images;
comparing the training index value with the target training data, and according to an instantaneous comparison result between the training index value and the target training data, generating prompt information corresponding to the target muscle so as to guide the training object in real time to perform target muscle training.

Preferably, the step of converting the training index values into visual feedback signals displayed in real time comprises:
constructing the visual feedback signal varying with time by using the training index values in the form of dynamic images and numerical readings, wherein, each frame of the dynamic image is used to represent the visual feedback signal of each time node, and the reading corresponding to the visual feedback signal is used to represent the degree of activation of the target muscle of the training object.

Preferably, the preset ultrasonic device comprises a probe;
accordingly, in the process of determining the training object based on the preset ultrasonic device, the positioning of the probe is guided by real-time feedback information; the feedback information is obtained by extracting at least one morphological parameter from skin layer, subcutaneous tissue layer and/or muscle layer and other soft tissues according to each frame of the ultrasonic images of the training object, and the morphological parameter is used to characterize the coupling and perpendicularity between the probe and the skin to ensure the consistency of probe placement.

Preferably, the step of according to an instantaneous comparison result between the training index value and the target training data, generating prompt information corresponding to the target muscle so as to guide the training object in real time to perform target muscle training, comprises:
according to the comparison result between the training index value and the target training data, determining whether the instantaneous relationship between the training index value calculated from the current ultrasonic image frame and the instaneous target training data meets a repetitive training condition, and generating the corresponding prompt information according to comparison results; wherein, a comparison calculation before generating the prompt information is carried out in real time based on each frame of the ultrasonic image;
expression of the prompt information comprises at least one of tactile, auditory and visual prompts.

Preferably, the ultrasonic image contains information related to the degree of muscle activation and is used to extract muscle characteristic parameters, the ultrasonic image comprises A-mode ultrasonic image, M-mode ultrasonic image, B-mode ultrasonic image, Doppler ultrasonic image or/and elastic ultrasonic image.

Preferably, the muscle characteristic parameters comprise biological information related to the degree of muscle activation, the biological information represents an activation state of the target muscle and characterizes the contraction function of the target muscle, the biological information comprises at least one of morphological parameters, physiological parameters, elastic parameters and image characteristic parameters;
accordingly the step of extracting muscle characteristic parameters of the target muscle from the selected regions of interest, comprises:
when the biological information is the morphological parameter, muscle thickness, muscle cross-sectional area, pinnate angle and muscle fiber length of the target muscle are extracted from the ultrasonic image by tracking and calculating the muscle displacement of the target muscle;
when the biological information is the physiological parameter, the blood flow velocity, blood flow direction, blood flow intensity and heart rate of the training object are extracted from the Doppler image;
when the biological information is the elastic parameter, Young's modulus, shear wave velocity, shear wave attenuation coefficient, shear modulus, bulk modulus, sound of speed, and ultrasonic attenuation coefficient are extracted from an echo signal and elastic image;
when the biological information is the image characteristic parameter, color feature parameters, texture feature parameters, shape feature parameters, and spatial relationship feature parameters are extracted from a two-dimensional digital image.

Preferably, the step of according to the muscle characteristic parameters, obtaining a training index value, representing a degree of activation of the target muscle of the training object during the exercise, comprises:
converting the muscle characteristic parameters into training index values through preset mathematical methods, and the training index values represent the degree of activation of the target muscle of the training object during the exercise;
   or
directly representing the training index value by the muscle characteristic parameters.

Preferably, a carrier of the visual feedback signal is a feedback device, and the feedback device is used for outputting the visual feedback signal; a type of the feedback device comprises a dial plat, a scale bar, an indicator light, a curve diagram and a mapping diagram.

Preferably, when the expression form of the visual feedback signal is the color coding change on the mapping diagram, the mapping diagram is constructed in the following way:
acquiring sampling regions in the selected region of interest, and determining the number and location of sampling regions in the selected region of interest;
collecting the muscle characteristic parameters in each sampling region respectively, and converting the muscle characteristic parameters into the training index values;
mapping the individual training index values to the image to be processed according to a specific spatial position, and the mapping diagram is constructed based on the image to be processed using a preset image post-processing technology, wherein a gray scale or color coding in the mapping diagram represents the amplitude of the visual feedback signal.

Preferably, the method further comprises:
at an end of the muscle training of the training object, generating a score based on an exercise performance of the target muscle of the training object in a contraction cycle, according to a deviation between a historical data of the training index value and the current target training data.

In addition, to achieve the above purposes, the present application also proposes a muscle training system, wherein, the muscle training system is a visual feedback system based on wireless ultrasonic imaging, and an ultrasonic device of the system comprises a host, a mobile terminal and at least one probe; the host comprises an ultrasonic unit, a feedback signal processing unit, a feedback device generating unit and a feedback reminder unit;
the probe is used to transmit ultrasonic wave to a training object and receive an echo signal of the training object;
the ultrasonic unit is used to construct ultrasonic images of the target muscle of the training object during contraction according to the echo signal;
the feedback signal processing unit is used to select regions of interest from ultrasonic image frames, extract muscle characteristic parameters of the target muscle from the selected regions of interest, and according to the muscle characteristic parameters, obtain a training index value, representing a degree of activation of the target muscle of the training object during the exercise;
the feedback device generating unit is used to convert the training index value into visual feedback signals displayed in real time, and synchronously display the visual feedback signal and the ultrasonic images on the mobile terminal;
the feedback reminder unit is used to compare the training index value with a target training data, and according to an instantaneous comparison result between the training index value and the target training data, generate prompt information corresponding to the target muscle so as to guide the training object in real time to perform target muscle training.

Preferably, the host further comprises a wireless transmission unit, which is used to realize bidirectional data transmission between the host and the mobile terminal.

Optionally, the mobile terminal is equipped with application software, and the application software on the mobile terminal is used to control an operation of each unit of the probe and the host;
the application on the mobile terminal is also used to perform post-processing of ultrasonic and visual feedback related data;
the application on the mobile terminal is also used to synchronously display ultrasonic images and visual feedback signals.

Preferably, a hardware combination of the system is an integrated structure; wherein, the probe and the ultrasonic unit, the feedback signal processing unit, the feedback device generating unit and the feedback reminder unit in the host are integrated into an all-in-one machine; the all-in-one machine performs data interaction with the mobile terminal through the wireless transmission unit.

Preferably, a hardware combination of the system is a split structure; wherein, the host is composed of the ultrasonic unit, the feedback signal processing unit, the feedback device generating unit, the feedback reminder unit, and the control unit in an integrated manner, and is wired with the probe through physical cables; the host is connected with the probe through physical cables or wirelessly through a wireless transmission unit; the host communicates data with the mobile terminal through the wireless transmission unit.

The technical scheme provided by the present application has the following beneficial effects: the present method first determining a training object and target training data on the basis of a preset ultrasonic device; then acquiring ultrasonic images of a target muscle of the training object during contraction process, and respectively selecting regions of interest from ultrasonic image frames; extracting muscle characteristic parameters of the target muscle from the selected regions of interest; according to the muscle characteristic parameters, obtaining a training index value, representing the degree of activation of the target muscle, of the training object during the exercise; converting the training index value into visual feedback signals displayed in real time, and synchronously displaying the visual feedback signals and the ultrasonic images; comparing the training index value with the target training data, and according to an instantaneous comparison result between the training index value and the target training data, generating prompt information corresponding to the target muscle so as to guide the training object in real time to perform target muscle training. Thus, a user can be guided to complete a targeted muscle activation task. The muscle training system provided by the present application is a visual feedback system based on wireless ultrasonic imaging. The ultrasonic device of the system includes a host, a mobile terminal and at least one probe. This portable system requires low professional skills and is easy to be understood, and is suitable for users who are not proficient in ultrasonic technology; during muscle training, users can visually and intuitively obtain feedback training performance at the first time, thereby improving the training effect and use experience of users.

### BRIEF DESCRIPTION OF THE DRAWINGS

Figure 1 is a flow diagram of the muscle training method for providing visual feedback by using ultrasonic imaging according to an embodiment of the present application;
Figure 2 is a schematic diagram for outputting a visual feedback signal using a curve diagram in an embodiment of the present application;
Figure 3 is a schematic diagram for outputting a visual feedback signal using a mapping diagram in an embodiment of the present application;
Figure 4 is a schematic diagram of training completion evaluation in an embodiment of the present application;
Figure 5a is a block diagram of an embodiment of the muscle training system of the present application;
Figure 5b is a block diagram of another embodiment of the muscle training system of the present application;
Figure 6 is the flow diagram involving completion evaluation in a muscle training method for providing visual feedback by using ultrasonic imaging according to another embodiment of the present application;
Figure 7 is a complete flow diagram of the muscle training method for providing visual feedback by using ultrasonic imaging according to the present application;
Figure 8 is another complete flow diagram of the muscle training method for providing visual feedback by using ultrasonic imaging according to the present application;
Figure 9 is a schematic diagram of a software user interface according to an embodiment of the present application;
Figure 10 is a structural diagram of the hardware combination according to an embodiment of the present application;
Figure 11 is a structural diagram of the hardware combination according to another embodiment of the present application;
Figure 12 is a structural diagram of the hardware combination according to one more embodiment of the present application.

The realization, functional features and advantages of the present application will be further described with reference to the attached drawings in combination with the embodiments.

### DETAILED DESCRIPTION OF THE PREFERRED EMBODIMENT

It should be understood that the specific embodiments described herein are only used to explain the present application, not to define the present application.

Understandably, traditional muscle training is empirically oriented and has low reliability. Trainers can only rely on subjective judgment and oral instructions to guide trainees and affect the training quality. In addition, the new model of using external equipment to assist muscle training has application prospects. On the one hand, using accelerometers, goniometers, torque sensors, far infrared cameras and other methods can only capture the external information of muscle movement, which cannot reflect the real activation state. In contrast, electromyography (EMG) and ultrasonic imaging can be used to characterize the muscle contraction function and collect the internal information of muscle movement.

The muscle training mode based on EMG biofeedback has preliminary application in clinical rehabilitation and scientific research. However, EMG signal is vulnerable to noise, environment and other disturbances, and the measurement accuracy is poor: it only acts on superficial muscle tissue, and it is difficult to refine to a single muscle, and it is impossible to distinguish the influence of surrounding muscle and deep muscle on target muscle (crosstalk phenomenon). Therefore, the above shortcomings restrict the application of EMG signal in muscle training.

Ultrasonic imaging has the advantages of real-time, non-invasive, non radiation, etc. It can observe the real activities of target muscle in the process of contraction and relaxation. Some studies have shown that, based on the quantitative relationship with mechanical properties, the physical characteristics of muscles (including elasticity and multiple morphological parameters) extracted from ultrasonic images can indicate the degree of muscle activation. However, the existing ultrasonic device is designed for medical purposes and operated by professional medical personnel. The movement state of muscles can only be monitored subjectively and cannot be expressed and recorded quantitatively. From the perspective of application, ultrasonic device is not only cumbersome and expensive, but also with a complex scanning process, and the ultrasonic image is obscure. From the perspective of user experience, traditional equipment includes the probe, the host, the display unit and other components, which are connected by cables; the holding mode of the probe and the lengthy cable affect the dynamic movement performance and action implementation (especially isotonic movement), which is reflected in the space restrictions on the range of motion of joints and limbs. Therefore, for businesses in the musculoskeletal direction, traditional ultrasound equipment is mainly used to qualitatively evaluate musculoskeletal diseases, and is not suitable for real-time muscle training.

The purpose of the present application is to provide a muscle training method and system for providing visual feedback by using ultrasonic imaging against the defects of the above technical scheme, which can measure the degree of activation of the muscle, convert it into a visual feedback signal and output it to the user in reverse, and guide the contraction of target muscle in real time according to the preset target threshold or target interval, improve the ability of motion control and ensure the repeatability of training. The system has the advantages of wireless transmission, miniaturization and portability, low professional requirements for users, and high cost efficiency.

The present application overcomes the defects of strong subjectivity, no quantitative reference standard and low consistency of traditional muscle training at present, and also overcomes the defects of low user friendliness of traditional ultrasonic equipment, in which only qualitative evaluation can be done.

The first embodiment (A muscle training method for providing visual feedback by using ultrasonic imaging)

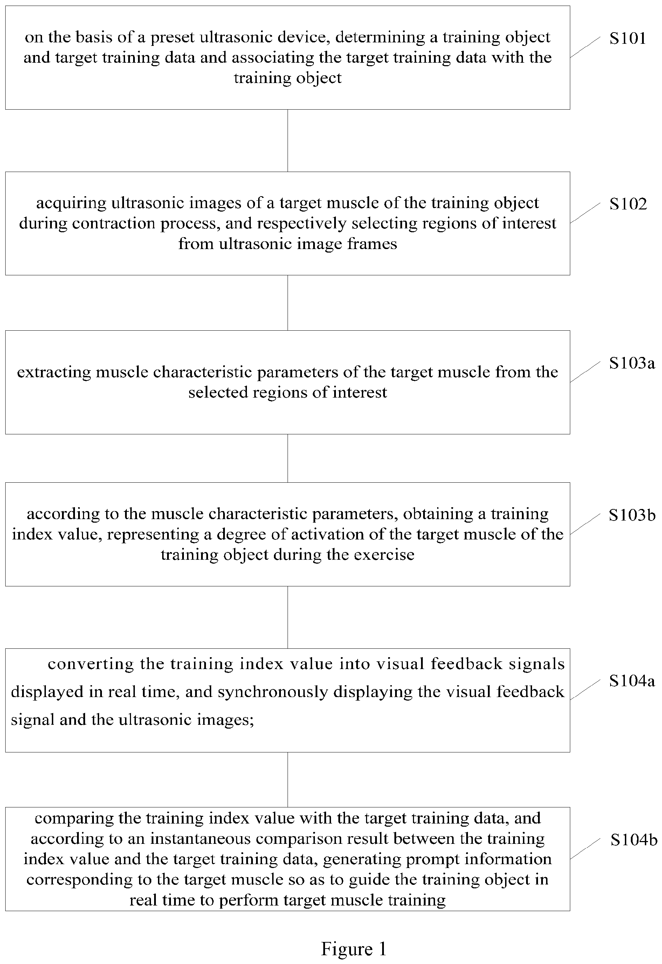

To achieve the above purpose, the embodiment of the present application provides a muscle training method for providing visual feedback by using ultrasonic imaging. Refer to Figure 1, which is the flow diagram of the muscle training method for providing visual feedback by using ultrasonic imaging.

It should be noted that the muscle training method for providing visual feedback by using ultrasonic imaging in this embodiment is implemented based on the muscle training system as the hardware equipment.

The muscle training system includes the preset ultrasonic device. The muscle training system is a visual feedback system based on wireless ultrasonic imaging. The ultrasonic device of the system at least includes a host, a mobile terminal and at least one probe.

In specific implementation, the probe is used to transmit ultrasonic wave to the training object and receive the echo signal of the training object. It should be noted that the training object can be understood as the human muscle tissue imaged by ultrasound, that is, the muscle tissue of the user who is using the preset ultrasonic device for muscle training, etc. The training object includes target muscle, non target muscle, skin, subcutaneous fat, fascia, ligament and joint and other soft tissue structures, as well as nearby blood vessels, nerves and bone and other non soft tissue structures.

The host executes the steps related to the muscle training method for providing visual feedback by using ultrasonic imaging:
Step S101: on the basis of a preset ultrasonic device, determining a training object and target training data and associating the target training data with the training object.

It should be noted that the target training data in this embodiment is a quantitative scheme to describe the activation target of target muscle, which can be preset as needed according to the user's training purpose, movement and personal situation (target training data can also be set by the user), and is mainly composed of the size and expression style of the training target value. Therefore, the host needs to associate the target training data with the training object.

The target training data can be a target threshold (i.e. single value K), and the target training data can also be a target interval (i.e. fluctuation within a certain range K ± k₁); the target training data (including target threshold, i.e. single value k, and target interval K ± k₁) can be expressed in any of the following ways: 1) constant, 2) periodic over time, or 3) random over time. It should be noted that the present application does not limit the numerical value and expression style of target training data.

Optionally, an implementation mode can be that in the process of determining the training object based on the preset ultrasonic device, the positioning of the probe is guided by real-time feedback information; the feedback information is obtained by extracting at least one morphological parameter from skin layer, subcutaneous tissue layer and/or muscle layer and other soft tissues according to each frame of the ultrasonic images of the training object, and the morphological parameter is used to characterize the coupling and perpendicularity between the probe and the skin to ensure a consistency of probe placement. In specific implementation, in this embodiment the probe is placed and fixed on the skin surface of the training target (focusing on target muscle) determined by the user in step S101. Fixing methods include manual holding, tape, strap, fixator, adhesive patch, etc.

Step S102: acquiring ultrasonic images of a target muscle of the training object during contraction process, and respectively selecting regions of interest from ultrasonic image frames.

With the implementation of the movement, the preset ultrasonic device will collect the ultrasonic image sequence of the movement of the muscle.

Specifically, the ultrasonic image contains information related to the degree of muscle activation and is used to extract muscle characteristic parameters, the ultrasonic image comprises A-mode ultrasonic image, M-mode ultrasonic image, B-mode ultrasonic image, Doppler ultrasonic image or/and elastic ultrasonic image.

Step S103a: extracting muscle characteristic parameters of the target muscle from the selected regions of interest.

It should be noted that, the muscle characteristic parameters comprise biological information related to the degree of muscle activation, the biological information represents an activation state of the target muscle and characterizes the contraction function of the target muscle, the biological information comprises at least one of morphological parameters, physiological parameters, elastic parameters and image characteristic parameters.

Specifically, in this embodiment digital, geometric transformation, image enhancement and other preprocessing will be done to the ultrasonic image sequences through image or signal processing technology. According to each ultrasonic image, the boundary of the target muscle tissue is detected and identified, and the region of interest (ROI) and the sampling area within the region of interest are determined; In the field of image processing, ROI (region of interest) is an image region selected from the image to be processed, which is the focus of image analysis.The area is delineated for further processing. Generally speaking, from the perspective of anatomy, the region of interest refers to a single muscle or an independent segment of a single muscle; from the perspective of imaging, the region of interest refers to an imaging section of the target muscle.

It should be noted that in this embodiment the number of regions of interest in each ultrasonic image is not limited, and each region of interest is independent of each other. In the delineated region of interest, at least one biological information describing the activation status of the target muscle and characterizing the contraction function is sampled quantitatively, which is called muscle characteristic parameters y; the sampling method can be carried out from multiple dimensions such as physiology, structure or function.

For a single region of interest, the location, the range and number of sampling regions are not limited in this embodiment; for a single sampling region, the type and number of muscle characteristic parameter y collected are not limited in this embodiment.

Generally, muscle characteristic parameters y include morphological parameters, physiological parameters, elastic parameters and image characteristic parameters. With regard to morphological parameters, the classic implementation is to extract at least one of the morphological parameters related to the tissue structure by tracking and calculating the muscle displacement; Preferably, muscle thickness from A-mode ultrasonic image, M-mode ultrasonic image or B-mode ultrasonic image can be extracted in this embodiment. Please note that for B-mode ultrasonic images, other non limiting morphological parameters include muscle cross-sectional area, pinnate angle, muscle fiber length, etc. Physiological parameters (referring to the hemodynamic characteristics related to muscle activation) are understood in a non restrictive way as the blood flow velocity, blood flow direction, blood flow intensity, heart rate, etc. extracted from the Doppler image. As for elastic parameters (referring to the physical characteristics related to biomechanical properties, which can indicate muscle hardness), in a non restrictive way, they are understood as Young's modulus, shear wave velocity, shear wave attenuation coefficient, shear modulus, volume modulus, sound of speed, ultrasonic attenuation coefficient, etc. extracted from echo signals and elastic images. As for image characteristic parameters, in a non restrictive way, they are understood as color features, texture features, shape features, spatial relationship features, etc. extracted from two-dimensional digital images.

On the other hand, the post-processing application of ultrasonic images can assist in the selection and fixation of the placement position of the probe: according to each ultrasonic image, the boundaries of the skin layer, subcutaneous tissue layer and/or muscle layer are detected and identified, and at least one morphological parameter in the soft tissue is extracted. In the process of placing the probe on the skin surface of the target muscle, the morphological information provides real-time feedback to characterize the coupling and perpendicularity between the probe and the skin. Its function is to ensure the consistency of probe position during repeated placement. This method determines the intrinsic parameters of soft tissue through ultrasonic images, which is helpful to improve the measurement reliability and reduce the error.

In specific implementation, when the biological information is the morphological parameter, muscle thickness, muscle cross-sectional area, pinnate angle and muscle fiber length of the target muscle are extracted from the ultrasonic image by tracking and calculating the muscle displacement of the target muscle;
when the biological information is the physiological parameter, the blood flow velocity, blood flow direction, blood flow intensity and heart rate of the training object are extracted from the Doppler image;
when the biological information is the elastic parameter, Young's modulus, shear wave velocity, shear wave attenuation coefficient, shear modulus, bulk modulus, sound of speed, and ultrasonic attenuation coefficient are extracted from an echo signal and elastic image;
when the biological information is the image characteristic parameter, color feature parameters, texture feature parameters, shape feature parameters, and spatial relationship feature parameters are extracted from a two-dimensional digital image.

Step S103b: according to the muscle characteristic parameters, obtaining a training index value, representing a degree of activation of the target muscle of the training object during the exercise.

In specific implementation, in this embodiment the muscle characteristic parameters are converted into training index values through preset mathematical methods, and the extracted muscle characteristic parameter y are converted into a quantitative training index in combination with the actual needs in the clinical or fitness fields. The training index value z represents the degree of the activation of the target muscle of the training object in the process of exercise, and reflects the training intensity; its function is to generate visual feedback signal.

Specifically, the training index value can be calculated by the muscle characteristic parameter y through a specific mathematical method, or the training index value can be directly represented by the muscle characteristic parameter y. For the training index value after mathematical processing, the non restrictive description form can be the change rate, ratio, difference value, or other calculated value after simple mathematical formula or complex mathematical model transformation.

Preferably, the training index value z is the result of normalization of muscle characteristic parameter y. It should be noted that the extraction of muscle characteristic parameter y and the calculation of training index value z are based on each frame of image in real time.

Step S104a: converting the training index value into a visual feedback signal displayed in real time, and synchronously displaying the visual feedback signal and the ultrasonic images.

In specific implementation, the host will construct the visual feedback signal varying with time by using the training index values in the form of dynamic images and readings, wherein, each frame of the dynamic image is used to represent the visual feedback signal of each time node, and the reading corresponding to the visual feedback signal is used to represent the degree of activation of the target muscle of the training object.

Understandably, the host converts the training index value z into visual feedback signals displayed in real time. Generally, the visual feedback signal consists of two elements: dynamic image and numerical reading, expressed in quantitative form, are used to indicate the current (current frame) muscle activation degree as the reference standard for visual feedback training; its carrier is called a feedback device, which is an indicating device, a medium for interaction with users, and is used to output visual feedback signals. The imaging frame rate of the ultrasonic image matches the operating frame rate of the feedback device. Preferably, the visual feedback signal is synchronously displayed with the traditional ultrasonic image for user observation.

It should be noted that the carrier of the visual feedback signal is a feedback device, and the feedback device is used to output the visual feedback signal; this embodiment does not limit the type of the feedback device and the display form of the visual feedback signal. The type of the feedback device is based on one-dimensional or two-dimensional structure, and the type of the feedback device includes a dial plat, a scale bar, an indicator light, a curve diagram, a mapping diagram, etc; The dial plat, the scale bar and the indicator light are one-dimensional feedback devices; the curve diagram and the mapping diagram are two-dimensional feedback devices.

Specifically, relative to the feedback device, the implementation of the display form of the visual feedback signal includes the curve amplitude change on the curve diagram, the pointer change on the dial plat, the light source brightness change on the indicator, the color or amplitude change on the scale bar, the color coding change on the mapping diagram, and so on.

When the expression form of the visual feedback signal is the color coding change on the mapping diagram, the mapping diagram is constructed in the following way:
acquiring sampling regions in the selected region of interest, and determining the number and location of sampling regions in the selected region of interest;
collecting the muscle characteristic parameters in each sampling region respectively, and converting the muscle characteristic parameters into the training index values;
mapping the individual training index values to the image to be processed according to a specific spatial position, and the mapping diagram is constructed based on the image to be processed using a preset image post-processing technology, wherein a gray scale or color coding in the mapping diagram represents the amplitude of the visual feedback signal.

Understandably, the mapping diagram represents the spatial distribution of muscle activation degree in different colors or grayscales, and provides visual feedback signals to users by using real-time changes in color coding. The specific way to construct the mapping diagram is to map the training index value z containing spatial position information to a one-dimensional or two-dimensional image through image post-processing methods such as interpolation and fitting, and display it in color or gray scale. Generally, the mapping diagram is also equipped with a scale bar, which uses color gradient and reading range to explain the intensity value coding of the mapping diagram.

Step S104b: comparing the training index value with the target training data, and according to an instantaneous comparison result between the training index value and the target training data, generating prompt information corresponding to the target muscle so as to guide the training object in real time to perform target muscle training.

In specific implementation, according to the comparison result between the training index value and the target training data, the preset ultrasonic device determines whether the instantaneous relationship between the training index value calculated from the current frame ultrasonic image and the target training data meets a repetitive training condition, and generates the corresponding prompt information according to a determination result; wherein, a comparison calculation before generating the prompt information is carried out in real time based on each frame of the ultrasonic image;
the expression of the prompt information comprises at least one of tactile, auditory and visual prompts.

Understandably, the preset ultrasonic device will compare the training index value z with the target training data K or K ± k₁ in real time based on each ultrasonic image, and give an alarm prompt according to the comparison result, reminding the user to accurately control the degree of activation of the target muscle.

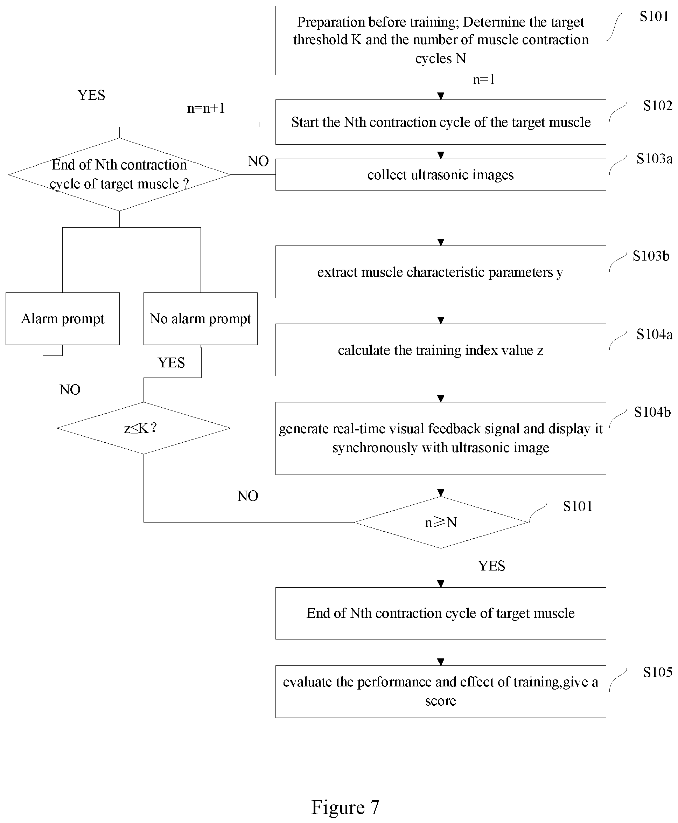

When the target training data is the target threshold k, refer to Figure 7, if the training index value z does not exceed the target threshold (that is, z ≤ K), there is no alarm prompt; if the training index value exceeds the target threshold (that is, z>K), the mobile terminal will output an instant alarm prompt to remind the user that effective activation has been achieved. The expression of the alarm prompt includes visual, auditory and tactile forms. After recording and completing the first contraction cycle of the target muscle, use the method of circulation until the Nth contraction cycle is completed.

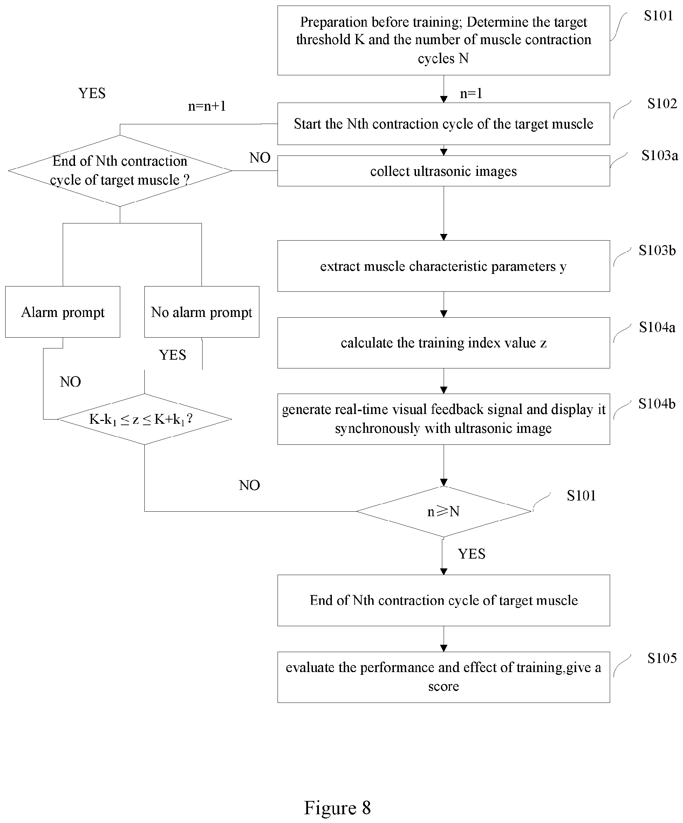

When the target training data is the target interval K ± k₁, refer to Figure 8 and compare the training index value z with the target interval K ± k₁: if the training index value is not within the target interval (that is, z>K+k₁ or z<K-k₁), the continuous alarm prompt will be output to remind the user that the degree of activation is not effective; if the training index value z is within the target interval (i.e. K-k₁ ≤ z ≤ K+k₁), the alarm will stop.

In addition, it should be noted that in another implementation mode, the corresponding functions of the steps S103a, S103b, S104a and S104b are performed by the mobile terminal; That is to say, the mobile terminal is equipped with application software, which is used to realize the operation control of the probe and the host of the preset ultrasonic device; moreover, the mobile terminal is also used to perform post-processing of ultrasonic and visual feedback related data, and synchronously display ultrasonic images and visual feedback signals.

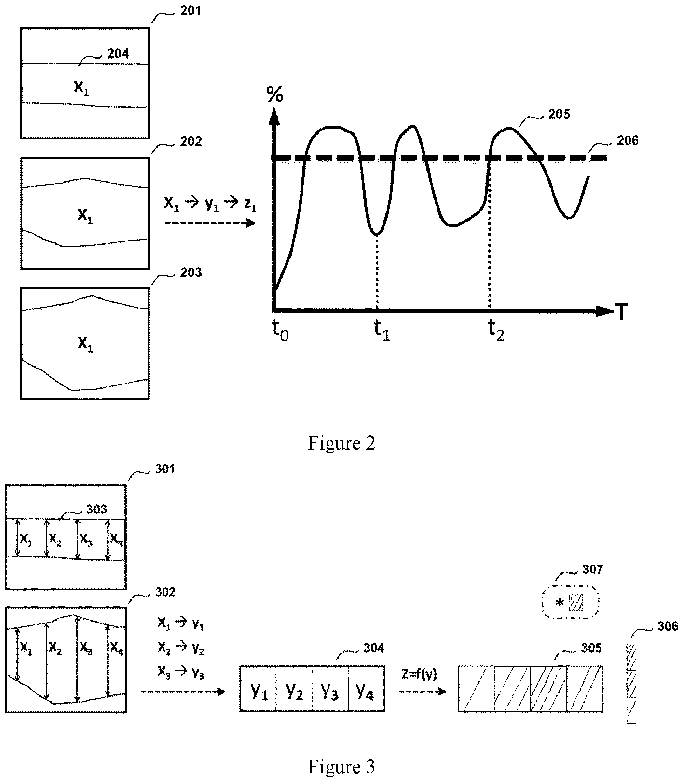

Refer to Figure 2, which is the dynamic curve diagram on the curve diagram when the type of the feedback device is two-dimensional curve diagram. In Figure 2, this implementation correspondingly selects the cross-sectional area as the muscle characteristic parameter, the ratio as the training index value, and the curve amplitude as the signal value . When the muscle is in the relaxed state (t0) 201 and the contractive states (t1) 202 and (t2) 203 of different frames, the change of the degree of activation will lead to the difference of the cross-sectional area. The region of interest 204 contains a single sampling area X1. According to the ultrasonic image of the corresponding frame, the cross-sectional area value y1 at different times is collected. Set the cross-sectional area value under the relaxed state 201 as the baseline, and use the ratio formula z=(y_{contraction}/yᵣₑₗₐₓₐₜᵢₒₙ) × 100% for the calculation and generation of the dynamic curve 205 of training index changing. The curve diagram is also equipped with a constant target threshold 206 for reference, which is the target training data preset by the user in step S101.

Referring to Figure 3, when the type of the feedback device is a one-dimensional mapping diagram, the visual feedback signal is displayed by the gray scale change on the mapping diagram (the gray scale change in Figure 3 is displayed by the shaded area). In this example, thickness is selected as the muscle characteristic parameter, thickness growth rate as the training index, and gray scale coding is used to represent the signal value. When the muscle is in the relaxed state (t0) 301 and the contractive state (t1) 302 of a frame, there is a significant difference in thickness; when the muscle is in the contractive state (t1) 302, the thickness at different positions also varies. With the change of the degree of activation , the thickness also changes correspondingly. The region of interest 303 includes several sampling regions arranged along the long axis of the target muscle, namely X1, X2, X3, X4. The muscle characteristic parameters y1, y2, y3 and y4 collected in each sampling area include the following information: activation status information (indicated by the thickness in this example) and position information (indicating one-dimensional spatial distribution), and form a single value mapping diagram 304. Set the thickness value under the relaxed state (t0) 301 as the baseline, calculate the thickness difference of each sampling area in each contraction image, and use the growth rate formula z=((y_{contraction} - yᵣₑₗₐₓₐₜᵢₒₙ)/yᵣₑₗₐₓₐₜᵢₒₙ) × 100% to obtain the real-time percentage change value, and this training index can represent the muscle deformation ability relative to the relaxed state. A one-dimensional gray scale mapping diagram 305 is constructed using mapping coding technology: indicate the degree of activation of different positions on the target muscle at time t₁; it is equipped with a scale bar 306 representing the numerical code of growth rate for interpretation and a target threshold 307 for reference.

The technical scheme provided by the embodiment has the following beneficial effects: the present method first determining a training object and target training data on the basis of a preset ultrasonic device; then acquiring ultrasonic images of a target muscle of the training object during contraction process, and respectively selecting regions of interest from ultrasonic image frames; extracting muscle characteristic parameters of the target muscle from the selected regions of interest; according to the muscle characteristic parameters, obtaining a training index value, representing the degree of activation of the target muscle, of the training object during the exercise; converting the training index value into visual feedback signals displayed in real time, and synchronously displaying the visual feedback signal and the ultrasonic images; comparing the training index value with the target training data, and according to an instantaneous comparison result between the training index value and the target training data, generating prompt information corresponding to the target muscle so as to guide the training object in real time to perform target muscle training. Thus, a user can be guided to complete an assigned muscle activation task. For example, only the deep muscle is contracted and the shallow muscle is maintained at the minimum amplitude/non contraction, maximum voluntary contraction (MVC), 50% MVC, etc. This scheme requires low professional skills, is portable and easy to understand, and is suitable for users who are not proficient in ultrasonic technology. During muscle training, users can visually and intuitively obtain feedback training performance at the first time, thereby improving the training effect and use experience of users. This method extends the application scenario of ultrasonic imaging technology to the field of muscle training related to personal fitness and rehabilitation, not limited to clinical disease diagnosis.

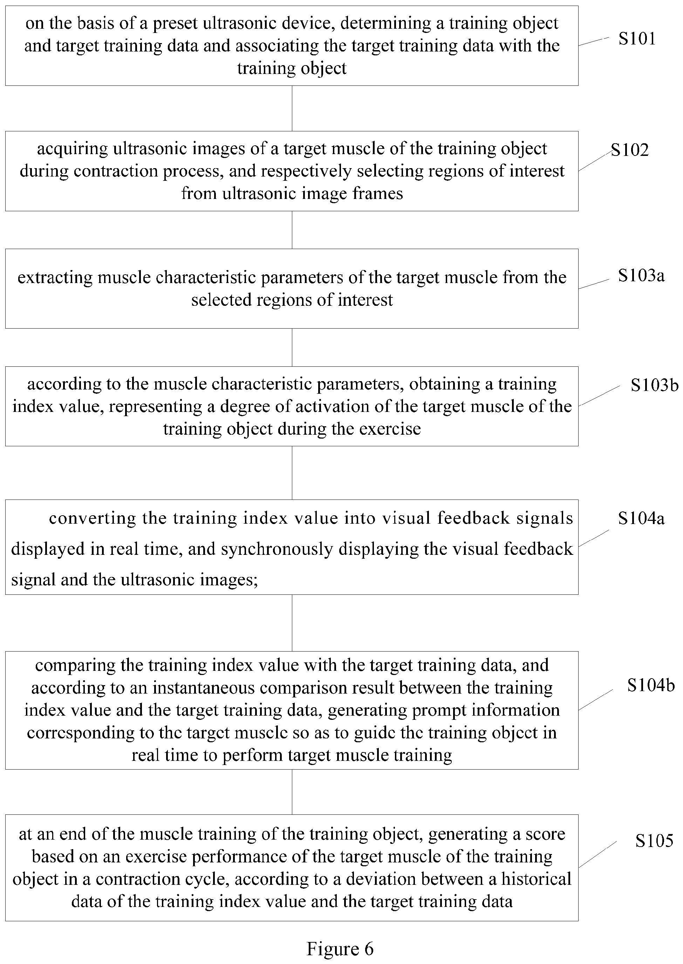

Further, referring to Figure 6, in another embodiment, after step S104b, the method also comprises:
Step S105: at an end of the muscle training of the training object, generating a score based on an exercise performance of the target muscle of the training object in a contraction cycle, according to a deviation between a historical data of the training index value and the current target training data.

In the specific implementation, the preset ultrasonic device generates a score based on the exercise performance of the target muscle of the training object in the contraction cycle. The completion evaluation can be divided into immediate evaluation and long-term evaluation.

Immediate evaluation: at the end of this training, combined with the past performance of the target muscle in the several contraction cycles, according to the deviation relationship with the preset target value (can be discrete or continuous), the comprehensive score is calculated and the training performance is evaluated retrospectively. Generally, the score is calculated by standard deviation, variance, mean square error and other statistical methods.

Long term evaluation: at the end of several training (or a training cycle) or periodically, measure the muscle characteristic parameters that can reflect the objective physical state of the target muscle, obtain its long-term change data over time, and track and evaluate the training effect. Generally, the muscle characteristic parameters include morphological parameters, elastic parameters, physiological parameters and image characteristic parameters collected from ultrasonic images. Preferably, Young's modulus is the core criterion.

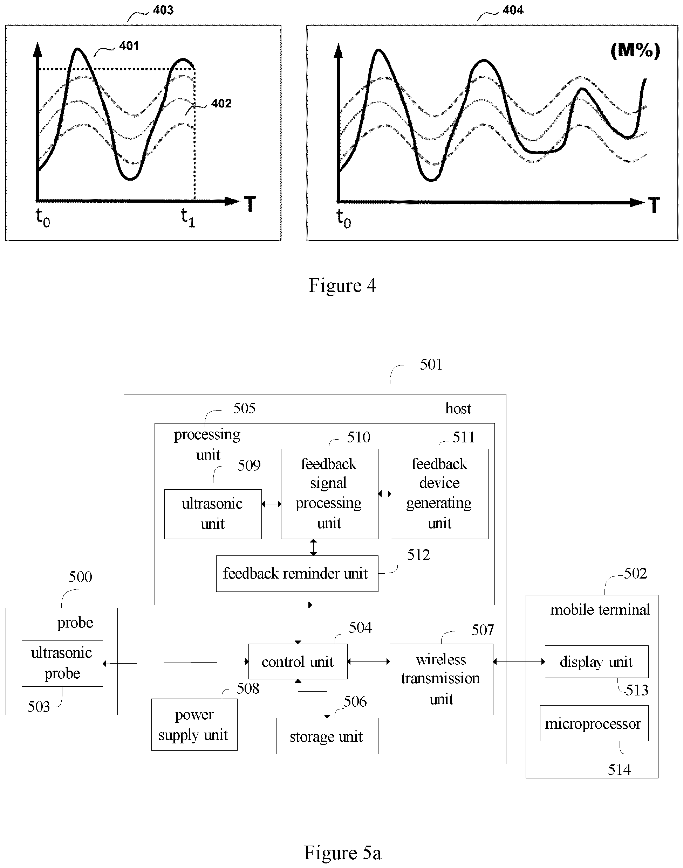

Referring to Figure 4, Figure 4 is an example of a specific implementation of training completion evaluation. As shown in curve diagram 403, this embodiment uses the curve diagram as the feedback device. The real-time change of dynamic curve 401 is used to represent the value of the visual feedback signal, and a periodically changing target interval 402 is provided for reference. At time t₁ (in the second contraction cycle), the amplitude of the curve represents the current reading of the degree of activation of the muscle. The curve diagram 404 shows the end time of the training, and the data of the past contraction cycle is visualized. Based on the degree of coincidence between the dynamic curve and the target interval, the score is M%.

The beneficial effect of this embodiment is to comprehensively evaluate the performance of contraction function based on the retrospective data of the muscle. The scores obtained can be used to formulate and adjust training programs, grade training performance, track records, monitor progress, judge prognosis, etc.

### The second embodiment (muscle training system )

Further, the present application also proposes an embodiment of a muscle training system. With reference to Figure 5a, Figure 5a is the structural block diagram of the muscle training system, which is a visual feedback system based on wireless ultrasonic imaging. The hardware of the ultrasonic device of the system includes a host 501, a probe 500 and a mobile terminal 502.

The probe 500 is used for transmitting ultrasonic wave to the training object and receiving the echo signal of the training object; the probe 500 consists of a single piezoelectric transducer array element arranged in a lattice or several piezoelectric transducer arrays arranged in a linear, convex or two-dimensional matrix to form the ultrasonic probe 503. The ultrasound probe 503 is responsible for transmitting ultrasound to the target muscle and receiving echo signals; Generally, it can support multiple imaging modes, including A-type, M-type, B-type, Doppler and elastic ultrasonic imaging. It should be noted that this embodiment does not limit the number of probes in a single system. In another embodiment, a single system is equipped with at least one probe connected, or multiple probes can be connected to the host at the same time. Multiple probes within a single system can be of the same/different size, type, and/or attribute. Due to the inherent limitations of the existing ultrasonic imaging technology, the scanning scope usually only covers a single muscle or a single segment of a single muscle. The embodiment provides an array, which relates to a multi unit probe. Its beneficial effect is that a single system can image a complete whole muscle or multiple muscles to meet the purpose of simultaneous training of the whole muscle or multiple muscles.

The host 501 includes a control unit 504, a processing unit 505, a storage unit 506, a wireless transmission unit 507, and a power supply unit 508. The control unit 504 is responsible for coordinating the functions and command transmission of each unit.

The processing unit 505 is composed of an ultrasonic unit 509, a feedback signal processing unit 510, a feedback device generating unit 511, and a feedback reminder unit 512; the ultrasonic unit 509 includes transmission/reception switch, beamformer, preamplifier, filter, AD converter, signal processor, image reconstruction device and other components, and is responsible for constructing traditional ultrasonic images, including A-type, M-type, B-type, Doppler image or elastic image. The ultrasonic unit 509 is used to construct ultrasonic images of the target muscle of the training object during contraction according to the echo signal

The feedback signal processing unit 510 is used to select regions of interest from ultrasonic image frames, extract muscle characteristic parameters of the target muscle from the selected regions of interest, and according to the muscle characteristic parameters, obtain a training index value, representing a degree of activation of the target muscle of the training object during the exercise.

Specifically, the feedback signal processing unit 510 includes a variety of signal/image analysis technologies such as identification, focusing, matching, segmentation, feature calculation, etc. Its functions include: 1) preprocessing images based on continuous ultrasonic images. 2) determining the region of interest and the sampling region based on each frame of image. 3) based on the selected region of interest, collecting the muscle characteristic parameter y. 4) through mathematical processing, calculating the training index value z by using the muscle characteristic parameter y.

The feedback device generating unit 511 is used to convert the training index value into visual feedback signals displayed in real time, and synchronously display the visual feedback signal and the ultrasonic images on the mobile terminal 502.

Specifically, the functions of the feedback device generating unit 511 include: 1) constructing a feedback device based on the training index value z to provide a visual feedback signal to the user in the form of a dynamic image and a reading display; 2) as for the feedback device is the embodiment of the mapping diagram, the work also includes the composition, superposition and fusion, and synchronization of the color mapping diagram.

The feedback reminder unit 512 is used to compare the training index value z with a target training data (K or K±k₁) , and according to an instantaneous comparison result between the training index value and the target training data, generate prompt information corresponding to the target muscle so as to guide the training object in real time to perform target muscle training.

In addition, it should be noted that the feedback signal processing unit 510, the feedback device generating unit 511 and the feedback reminder unit 512 can be either hardware based or software based, and their settings are not limited to the host 501; optionally, the corresponding functions of the feedback signal processing unit 510, the feedback device generating unit 511 and the feedback reminder unit 512 can also be performed by the mobile terminal 502. That is to say, the mobile terminal is equipped with application software, and the application software on the mobile terminal is used to realize the operation control of each unit of the probe and the host. It is also used to perform post-processing of the data related to the ultrasonic and visual feedback. It is also used to synchronously display ultrasonic images and visual feedback signals.

Software execution code and processing algorithm are stored in the storage unit 506, which is responsible for caching and packaging ultrasonic images and data related to visual feedback.

The wireless transmission unit 507 is used to realize bidirectional data transmission between the host and the mobile terminal. The wireless connection between the host 501 and the mobile terminal 502 is realized. Sending the data temporarily stored in the storage unit 506 after being processed by the processing unit 505 to the mobile terminal 502 by means of wireless transmission; on the other hand, the wireless transmission unit 507 can also receive control instructions sent by the mobile terminal 502. The control commands include the adjustment of ultrasonic imaging parameters, the setting of training targets, the selection of sampling mode and the type of the feedback signal, etc. Wireless communication technologies include WiFi, Bluetooth, UWB ultra bandwidth, ZigBee, 4G technology or 5G technology. Imaging data and control commands are transmitted via wireless communication, which not only enables information interaction with cloud database, but also promotes hardware miniaturization and integration.

The power supply unit 508 contains batteries and related circuits and is responsible for supplying power to the probe 500 and the host 501. Charging methods include wireless charging and wired charging.

It should be noted that the beneficial effect of this embodiment is that compared with the existing large-scale ultrasonic imaging system, the solution of the embodiment has low profile and low-cost, especially suitable for private consumers. This embodiment extends the application scenario of ultrasonic equipment to the field of muscle training related to personal fitness and rehabilitation, not limited to clinical disease diagnosis. In addition, the mobile terminal 502 has a display unit 513 and a microprocessor 514, including a smart phone, a desktop computer, a notebook, a tablet computer, a smart TV, and the like. The application software (App) running on the mobile terminal 502 is a platform for interaction with the user end. The microprocessor 514 is responsible for post-processing data and executing user operation instructions to control the operation of each unit of the probe and the host, and the display unit 513 is responsible for displaying ultrasonic images and visual feedback signals. It should be noted that ultrasonic images and data related to visual feedback can be downloaded locally to mobile terminal 502 for reference or post-processing, or uploaded to cloud database 603.

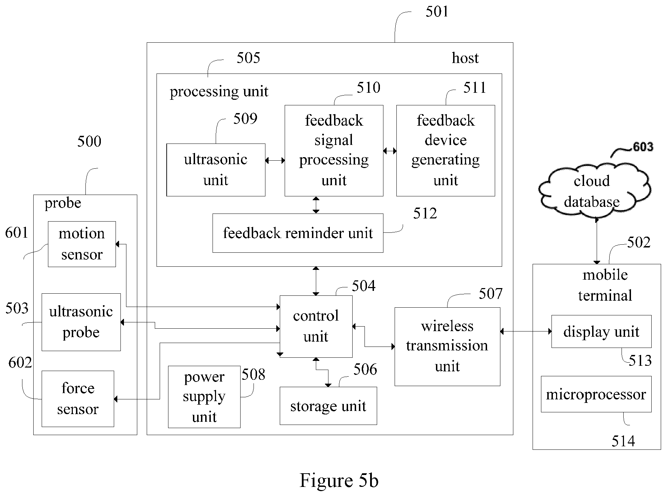

Optionally, referring to Fig. 5b, a motion sensor 601 and a force sensor 602 are added to the probe 500. The motion sensor 601 is composed of an inertial measurement unit or a plurality of integrated accelerometers and gyroscopes. It is coupled to the ultrasonic probe 503, packaged into a probe 500, and attached to a certain area of the skin surface of the trained limb/body for multi axis motion tracking: recording angular velocity, acceleration, velocity, angle and other dynamic motion data. This additional information can be used to calculate the position and movement posture of the probe 500 in the three-dimensional space, which can be used as an important reference for the standardized implementation of fitness movements.

The at least one force sensor 602 is coupled to the ultrasonic probe 503 and can measure the pressure applied on the contact surface of the probe 500. This information can evaluate and standardize the fixation effect of the probe 500 on the skin surface of the target muscle, that is, whether it is in full contact with the appropriate pressure and whether it is placed vertically. Poor probe fixation will negatively affect the imaging quality, and even cause it to loosen and fall during muscle contraction. As the target audience of this embodiment is applicable to users lacking professional skills, not limited to medical personnel, additional mechanical information can participate in the quality control of probe fixation, improve consistency, and facilitate standardized operations. It is worth noting that the quality control of the probe fixation can also be conducted under the guidance of image related feedback information; the feedback information is obtained by post-processing the ultrasonic image (as described in the first embodiment). It should be noted that the additional collected mechanical and motion information will be bundled with each ultrasonic image in chronological order and sent to the processing unit 505 for synchronous processing.

In order to make full use of the advantages of the present application based on wireless communication, portability and the like, the mobile terminal 502 can exchange data with the cloud server. The cloud database 603 is used for the storage, retrospective analysis and sharing of the data. A big data workstation can also be set up on the ECS to perform cloud computing.

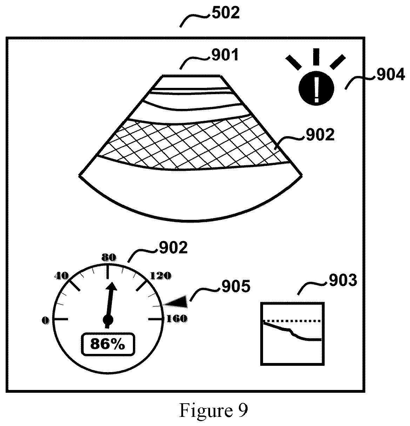

Further, referring to Fig. 9, Fig. 9 is an embodiment of the software part of the present application. The software user interface consists of an ultrasonic image 901, a feedback device 902, a pressure indicator 903, and a feedback reminder indicator 904, which is displayed on the mobile terminal 502. This embodiment takes the acquisition of B-mode ultrasonic images as an example to illustrate, and selects target muscle 902 as the region of interest: extract training related parameters y and z, and convert them into visual feedback signals. In this example, the dial plat is selected as the feedback device 902. The visual feedback signal is displayed by the changes of the pointer on the dial plat. The reading and the pointer pointing are used to indicate the instantaneous degree of activation of the muscle, and a target threshold value 905 is provided for reference. The pressure indicator 903 is used to monitor the fixed instantaneous state of the probe and feed back the pressure information to the user. The feedback reminder indicator 904 sends out an alarm to prompt the instantaneous relationship between the degree of activation of the muscle calculated in the current frame and the target threshold. For spatial layout, the feedback device and ultrasonic image are independently set on the user interface. Another implementation scheme is to superimpose visual feedback signals on the basis of ultrasonic images. Preferably, the result of superposition and fusion display of mapping diagram and ultrasonic image is: in the same region of interest, the shape information and information of the degree of activation of the muscle are displayed at the same time.

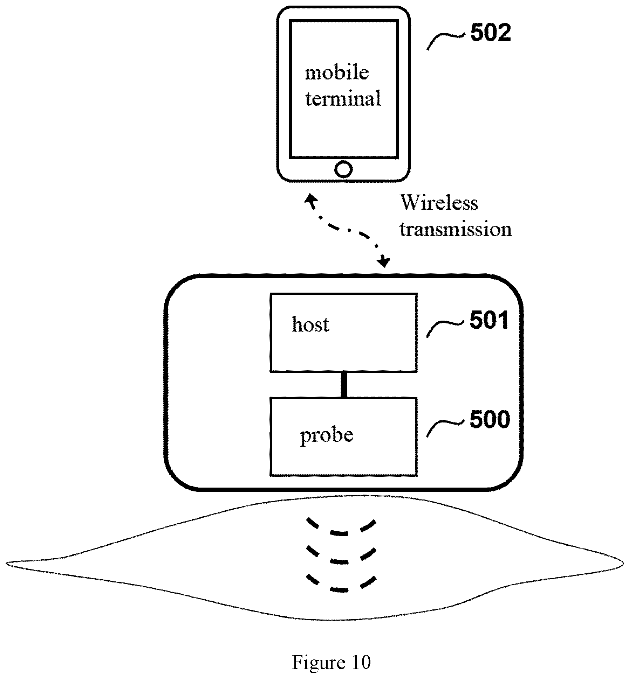

Referring to Figure 10, in this embodiment, the hardware combination of the system can be an integrated structure; wherein, the probe 500 and the ultrasonic unit, the feedback signal processing unit, the feedback device generating unit and the feedback reminder unit in the host 501 are integrated into an all-in-one machine. The all-in-one machine performs data interworking with the mobile terminal 502 through the wireless transmission unit. It can be understood that this integration mode has no trouble of cables, all components are connected wirelessly, and the overall structure is simple and compact, which is the optimal concept of the present application. This technical assumption is applicable to all dynamic and static muscle working modes (i.e. isometric contraction, isotonic contraction, isokinetic contraction), especially solving the problem of space obstruction caused by repeated isotonic movement of limbs and joints by cables.

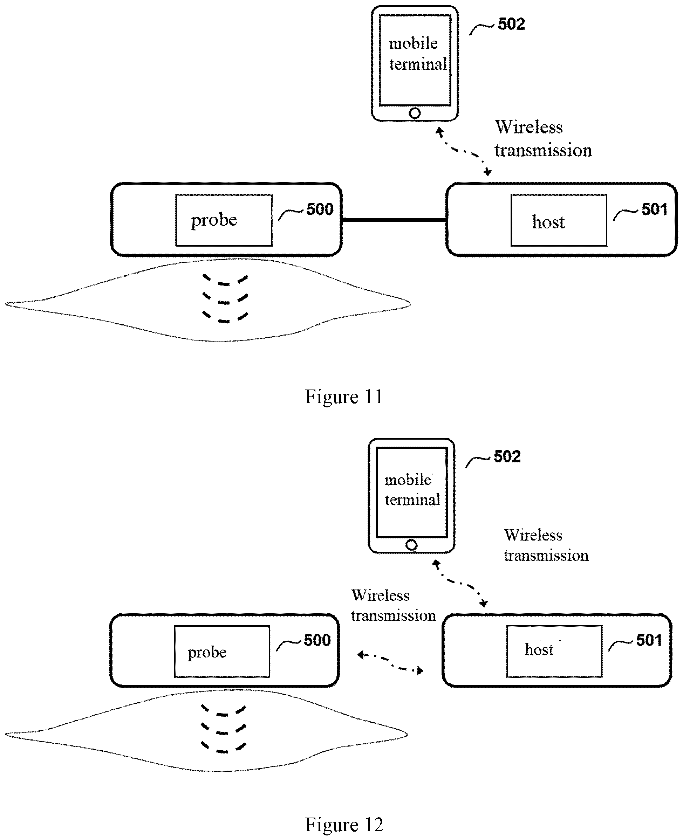

In this embodiment, the hardware combination of the system can also be a separate structure; with reference to Figure 11, the host is composed of the ultrasonic unit, the feedback signal processing unit, the feedback device generating unit, the feedback reminder unit, and the control unit in an integrated manner, and is wired with the probe through physical cables; the host communicates data with the mobile terminal through the wireless transmission unit. Referring to Figure 12, in another embodiment, the host is composed of the ultrasonic unit, the feedback signal processing unit, the feedback device generating unit, the feedback reminder unit, and the control unit in an integrated manner, and is wirelessly connected with the probe and the mobile terminal through the wireless transmission unit; the probe is also equipped with a wireless transmission unit for data exchange. Understandably, in actual operation, the two separate ways use host to share the weight and volume of the probe, which is conducive to its fixation on the skin surface; the separate host is convenient to be carried. This technical assumption is relatively easy to implement.

The beneficial effect of the above embodiments of Figures 10, 11 and 12 is that the hardware combination can minimize the limitation of the cable and probe handheld mode on muscle training (especially isotonic movement); providing a basis for the design and development of wearable ultrasonic probes.

In order to better understand the muscle training method and system providing visual feedback by using ultrasonic imaging provided by the embodiments of the present application, the complete workflow of the above embodiment scheme is further described:
For the first embodiment of the complete workflow, refer to Figure 7, step 1 (S101): preparation before training. The specific contents include setting the target training data K or K ± k₁ in advance, determining the target muscle to be trained, determining the number of the contraction cycles N of the muscle, determining the training related parameters y and z, selecting the type of feedback signal, fixing the ultrasonic probe, etc. Step 2 (S102): Start the first contraction cycle of the target muscle and collect ultrasonic images. Step 3 (S103a): extract muscle characteristic parameters y. Step 4 (S103b): calculate the training index value z. Step 5 (S104a): generate real-time visual feedback signal and display it synchronously with ultrasonic image. Step 6 (S104b): remind the feedback based on target threshold. The technical solution to achieve human-computer interaction is to use feedback reminder to guide training: provide feedback reminder unit 512, and compare the training index value y with the preset target threshold value K. If the training index value does not exceed the target threshold value (i.e. z ≤ K), there is no alarm prompt; if the training index value exceeds the target threshold value (i.e. z>K), the mobile terminal 502 will output an instant alarm prompt to remind the user that the degree of activation level is effective. Unlimitedly, the expression of alarm prompt includes visual, auditory and tactile forms. After recording and completing the first contraction cycle of the target muscle, use the method of circulation until the Nth contraction cycle is completed. Step 7 (S105): evaluate the performance and effect of training: review the N contraction cycles in the past, evaluate the training completely and give a score.

For the second embodiment of the complete workflow, refer to Figure 8. Step 6 (S104b) can also be implemented through another technical solution. In addition, other steps are similar to those in the first embodiment above, so we will not repeat them. Step 6 (S104b): remind the feedback based on target range. The feedback reminding unit 512 is provided to compare the training index value y with the preset target interval K ± k₁: if the training index value is not within the target interval (i.e. z>K+k₁ or z<K-k₁), the mobile terminal 102 will output a continuous alarm prompt to remind the user that the training index value y is not in the effective degree of activation; if the training index value is within the target range (i.e. K-k₁ ≤ z ≤ K+k₁), the alarm will stop.

It should be noted that in this application, the terms "include", "comprise" or any other variant thereof are intended to cover non exclusive inclusion, so that a process, method, article or system that includes a series of elements not only includes those elements, but also includes other elements that are not explicitly listed, or also includes elements inherent in such process, method, article or system. Without more restrictions, the element defined by the statement "including one..." does not exclude the existence of another identical element in the process, method, article or system that includes the element.

The serial number of the embodiments of the present application is only for description and does not represent the advantages and disadvantages of the embodiments.

Through the above description of the embodiments, those skilled in the art can clearly understand that the above embodiments can be implemented by means of software plus the necessary general hardware platform, of course, hardware, but in many cases the former is a better implementation. Based on this understanding, the technical solution of the present application in essence or the part that contributes to the prior art can be embodied in the form of a software product, which is stored in a storage medium (such as ROM/RAM, magnetic disc, optical disc) as described above, A plurality of instructions are included to enable a terminal device (which can be a mobile phone, a computer, a server, an air conditioner, or a network device) to execute the methods described in various embodiments of the present application.

The above is only the preferred embodiment of the present application, and does not limit the scope of the present application. Any equivalent structure or equivalent process transformation made by using the contents of the description and the attached drawings of the present application, or directly or indirectly applied in other related technical fields, are similarly included in the scope of patent protection of the present application.

## Claims

1. A muscle training method for providing visual feedback by using ultrasonic imaging, wherein, the method comprises:
on the basis of a preset ultrasonic device, determining a training object and target training data and associating the target training data with the training object;
acquiring ultrasonic images of a target muscle of the training object during contraction process, and respectively selecting regions of interest from ultrasonic image frames;
extracting muscle characteristic parameters of the target muscle from the selected regions of interest;
according to the muscle characteristic parameters, obtaining a training index value, representing a degree of activation of the target muscle of the training object during the exercise;
converting the training index value into visual feedback signals displayed in real time, and synchronously displaying the visual feedback signal and the ultrasonic images;
comparing the training index value with the target training data, and according to an instantaneous comparison result between the training index value and the target training data, generating prompt information corresponding to the target muscle so as to guide the training object in real time to perform target muscle training.

2. The method according to claim 1, wherein, the step of converting the training index values into visual feedback signals displayed in real timecomprises:
constructing the visual feedback signal varying with time by using the training index values in the form of dynamic images and numerical readings, wherein, each frame of the dynamic image is used to represent the visual feedback signal of each time node, and the reading corresponding to the visual feedback signal is used to represent the degree of activation of the target muscle of the training object.

3. The method according to claim 1, wherein, the preset ultrasonic device comprises a probe;
accordingly, in the process of determining the training object based on the preset ultrasonic device, the positioning of the probe is guided by real-time feedback information; the feedback information is obtained by extracting at least one morphological parameter from skin layer, subcutaneous tissue layer and/or muscle layer and other soft tissues according to each frame of the ultrasonic images of the training object, and the morphological parameter is used to characterize the coupling and perpendicularity between the probe and the skin to ensure the consistency of probe placement.

4. The method according to claim 1, wherein, the step of according to an instantaneous comparison result between the training index value and the target training data, generating prompt information corresponding to the target muscle so as to guide the training object in real time to perform target muscle training, comprises:
according to the comparison result between the training index value and the target training data, determining whether the instantaneous relationship between the training index value calculated from the current ultrasonic image frame and the instaneous target training data meets a repetitive training condition, and generating the corresponding prompt information according to comparison results; wherein, a comparison calculation before generating the prompt information is carried out in real time based on each frame of the ultrasonic image;
expression of the prompt information comprises at least one of tactile, auditory and visual prompts.

5. The method according to claim 1, wherein, the ultrasonic image contains information related to the degree of muscle activation and is used to extract muscle characteristic parameters, the ultrasonic image comprises A-mode ultrasonic image, M-mode ultrasonic image, B-mode ultrasonic image, Doppler ultrasonic image or/and elastic ultrasonic image.

6. The method according to claim 1, wherein, the muscle characteristic parameters comprise biological information related to the degree of muscle activation, the biological information represents an activation state of the target muscle and characterizes the contraction function of the target muscle, the biological information comprises at least one of morphological parameters, physiological parameters, elastic parameters and image characteristic parameters;
accordingly the step of extracting muscle characteristic parameters of the target muscle from the selected regions of interest, comprises:
when the biological information is the morphological parameter, muscle thickness, muscle cross-sectional area, pinnate angle and muscle fiber length of the target muscle are extracted from the ultrasonic image by tracking and calculating the muscle displacement of the target muscle;
when the biological information is the physiological parameter, the blood flow velocity, blood flow direction, blood flow intensity and heart rate of the training object are extracted from the Doppler image;
when the biological information is the elastic parameter, Young's modulus, shear wave velocity, shear wave attenuation coefficient, shear modulus, bulk modulus, sound of speed, and ultrasonic attenuation coefficient are extracted from an echo signal and elastic image;
when the biological information is the image characteristic parameter, color feature parameters, texture feature parameters, shape feature parameters, and spatial relationship feature parameters are extracted from a two-dimensional digital image.

7. The method according to claim 1, wherein, the step of according to the muscle characteristic parameters, obtaining a training index value, representing a degree of activation of the target muscle of the training object during the exercise, comprises:
converting the muscle characteristic parameters into training index values through preset mathematical methods, and the training index values represent the degree of activation of the target muscle of the training object during the exercise;
or
directly representing the training index value by the muscle characteristic parameters.

8. The method according to one of claims 1 to 7, wherein, a carrier of the visual feedback signal is a feedback device, and the feedback device is used for outputting the visual feedback signal; a type of the feedback device comprises a dial plat, a scale bar, an indicator light, a curve diagram and a mapping diagram.

9. The method according to one of claims 1 to 7, wherein, when the expression form of the visual feedback signal is the color coding change on the mapping diagram, the mapping diagram is constructed in the following way:
acquiring sampling regions in the selected region of interest, and determining the number and location of sampling regions in the selected region of interest;
collecting the muscle characteristic parameters in each sampling region respectively, and converting the muscle characteristic parameters into the training index values;
mapping the individual training index values to the image to be processed according to a specific spatial position, and the mapping diagram is constructed based on the image to be processed using a preset image post-processing technology, wherein a gray scale or color coding in the mapping diagram represents the amplitude of the visual feedback signal.

10. The method according to claim 8, wherein, the method further comprises:
at an end of the muscle training of the training object, generating a score based on an exercise performance of the target muscle of the training object in a contraction cycle, according to a deviation between a historical data of the training index value and the current target training data.

11. A muscle training system, wherein, the muscle training system is a visual feedback system based on wireless ultrasonic imaging, and an ultrasonic device of the system comprises a host, a mobile terminal and at least one probe; the host comprises an ultrasonic unit, a feedback signal processing unit, a feedback device generating unit and a feedback reminder unit;
the probe is used to transmit ultrasonic wave to a training object and receive an echo signal of the training object;
the ultrasonic unit is used to construct ultrasonic images of the target muscle of the training object during contraction according to the echo signal;
the feedback signal processing unit is used to select regions of interest from ultrasonic image frames, extract muscle characteristic parameters of the target muscle from the selected regions of interest, and according to the muscle characteristic parameters, obtain a training index value, representing a degree of activation of the target muscle of the training object during the exercise;
the feedback device generating unit is used to convert the training index value into visual feedback signals displayed in real time, and synchronously display the visual feedback signal and the ultrasonic images on the mobile terminal;
the feedback reminder unit is used to compare the training index value with a target training data, and according to an instantaneous comparison result between the training index value and the target training data, generate prompt information corresponding to the target muscle so as to guide the training object in real time to perform target muscle training.

12. The system according to claim 11, wherein, the host further comprises a wireless transmission unit, which is used to realize bidirectional data transmission between the host and the mobile terminal.

13. The system according to claim 11 or 12, wherein, the mobile terminal is equipped with application software, and the application software on the mobile terminal is used to control an operation of each unit of the probe and the host;
the application on the mobile terminal is also used to perform post-processing of ultrasonic and visual feedback related data;
the application on the mobile terminal is also used to synchronously display ultrasonic images and visual feedback signals.

14. The system according to claim 12, wherein, a hardware combination of the system is an integrated structure; wherein, the probe and the ultrasonic unit, the feedback signal processing unit, the feedback device generating unit and the feedback reminder unit in the host are integrated into an all-in-one machine; the all-in-one machine performs data interaction with the mobile terminal through the wireless transmission unit.

15. The system according to claim 12, wherein, a hardware combination of the system is a split structure; wherein, the host is composed of the ultrasonic unit, the feedback signal processing unit, the feedback device generating unit, the feedback reminder unit, and the control unit in an integrated manner, and is wired with the probe through physical cables; the host is connected with the probe through a physical cable or wirelessly through a wireless transmission unit; the host communicates data with the mobile terminal through the wireless transmission unit.
